# EUROPÄISCHE PATENTSCHRIFT

(11) **EP 1 487 839 B1**
(45) Veröffentlichungstag und Bekanntmachung des Hinweises auf die Patenterteilung: **18.04.2007**
(21) Anmeldenummer: 03702175.5
(22) Anmeldetag: 09.01.2003
(51) Int. Cl.: C07D 491/06

(54) **VERFAHREN ZUR HERSTELLUNG VON NORGALANTHAMIN, IHREN ISOMEREN, SALZEN UND HYDRATEN**
METHODS FOR PRODUCING NORGALANTHAMINE, AS WELL AS ISOMERS, SALTS AND HYDRATES THEREOF
PROCEDES POUR LA PRODUCTION DE NORGALANTHAMINE, SES ISOMERES, SELS ET HYDRATES

(30) Priorität: 25.03.2002 AT 4632002
(43) Veröffentlichungstag der Anmeldung: 22.12.2004
(60) Teilanmeldung: 06008743.4
(73) Patentinhaber: Sanochemia Pharmazeutica Aktiengesellschaft, 1090 Wien (AT)
(72) Erfinder: TREU, Matthias, A-1220 Wien (AT); HIRNSCHALL, Manfred, A-1160 Wien (AT); FRÖHLICH, Johannes, A-1050 Wien (AT); CZOLLNER, Laszlo, A-2490 Ebenfurth (AT); KÄLZ, Beate, A-7035 Steinbrunn (AT); KÄLZ, Thomas, A-7035 Streinbrunn (AT); KÜHNHACKL, Peter, A-1170 Wien (AT); JORDIS, Ulrich, A-1180 Wien (AT)
(74) Vertreter: Beer, Manfred
(86) Internationale Anmeldenummer: PCT/AT2003/000007
(87) Internationale Veröffentlichungsnummer: WO 2003/080623

(56) Entgegenhaltungen:
- WO-A-01/74820
- WO-A-97/03987
- US-A- 5 958 903
- MARY A ET AL: "Selective N-Demethylation of Galanthamine to Norgalanthamine via a non classical Polonovski Reaction" TETRAHEDRON LETTERS, ELSEVIER SCIENCE PUBLISHERS, AMSTERDAM, NL, Bd. 38, Nr. 29, 21. Juli 1997 (1997-07-21), Seiten 5151-5152, XP004082968 ISSN: 0040-4039 in der Anmeldung erwähnt

## Beschreibung

Die Erfindung betrifft ein Verfahren zur Herstellung von Salzen des Norgalanthamin mit der allgemeinen Formel B 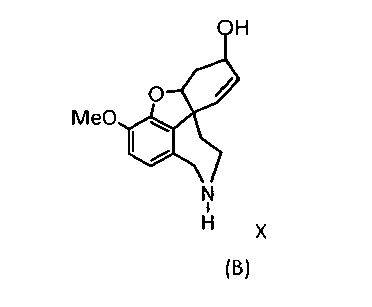 wobei X gleich HCl oder Oxalsäure ist.

Verbindungen mit der allgemeinen Formel A 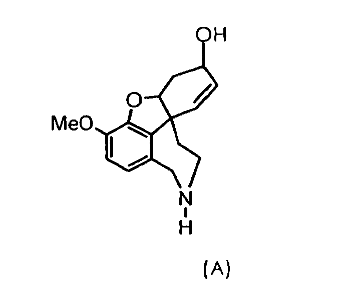 sowie deren Salze und Hydrate mit der allgemeinen Formel B 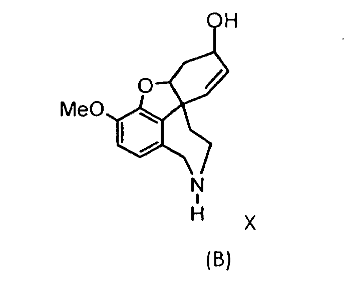 sind Schlüssel-Zwischenprodukte zur Herstellung von synthetischen Galanthaminderivaten, welche u.a. zur Behandlung von Erkrankungen des Zentralnervensystems (ZNS), wie Morbus Alzheimer, verwendet werden.

Verbindungen der allgemeinen Formel (A) und (B) sind bekannt und können beispielsweise aus Naturstoffen gewonnen werden, siehe:
Sener, Bilge; Konukol, Sakine; Kruk, Cornelis; Pandit, Upendra K. **Alkaloids from Amaryllidaceae. III. Alkaloids from the bulbs of Pancratium maritimum**. Nat. Prod. Sci. **1998**, *4,* 148 - 152
Eichhorn, Jorg; Takada, Takeshi; Kita, Yasuyuki; Zenk, Meinhart H. **Biosynthesis of the Amaryllldaceae alkaloid galanthamine**. Phytochemistry **1998**, *49*, 1037 - 1047
Almanza, Giovanna R.; Fernandez, Juan M.; Wakori, Edith W. T.; Viladomat, Francesc; Codina, Carles; Bastida, Jaume. **Alkaloids from Narcissus cv. Salome.** Phytochemistry **1996,** *43*, 1375 - 1378
Latvala, Anita; Oenuer, Mustafa A.; Goezler, Tekant; Linden, Anthony; Kivcak, Bijen; Hesse, Manfred. **Alkaloids** of **Galanthus elwesil.** Phytochemistry **1995,** *39*, 1229 - 1240.
Kreh, Mirko; Matusch, Rudolf. **O-Methyloduline** and **N-demethylmasonine, alkaloids from Narcissus pseudonarcissus.** Phytochemistry **1995,** *38*, 1533 - 1535
Bastida, Jaume; Viladomat, Francesc; Bergonon, Salvador; Fernandez, Juan marcos; Codina, Carles; Rubiralta, Mario; Quirion, Jean Charles. **Narcissus alkaloids.** Part 19. **Alkaloids** from **Narcissus leonensis.** Phytochemistry **1993,** *34*, 1656 - 1658
Weniger, B.; Italiano, L.; Beck, J.-P.; Bastida, J.; Bergonon, S.; Codina, C.; Lobstein, A.; Anton, R. **Cytotoxic activity of Amaryllidaceae alkaloids.** Planta Med. **1995,** *61*, 77 - 79
Bastida, J.; Viladomat, F.; Llabres, J. M.; Quiroga, S.; Codina, C.; Rubiralta, M. **Narcissus alkaloids.** Part **IX. Narcissus nivalis: a new source of galanthamine.** Planta Med. **1990**, *56*, 123 - 124
Kihara, Masaru; Koike, Tomomi; Imakura, Yasuhiro; Kida, Kiyoshi; Shingu, Tetsuro; Kobayashi, Shigeru. **Alkaloidal constituents of Hymenocallis rotata Herb. (Amaryllidaceae).** Chem. Pharm. Bull. **1987**, *35*, 1070 - 1075
Kobayashi, Shigeru; Ishikawa, Hideki; Kihara, Masaru; Shing, Tetsuro; Uyeo, Shojiro. **Isolation** of **N-demethylgalanthamine** from the bulbs of **Crinum asiaticum L**. **var. Japanicum Baker (Amaryllidaceae).** Chem. Pharm. Bull. **1976,** *24*, 2553 - 2555

Weiters ist es bekannt, Verbindungen der allgemeinen Formel A bzw. B synthetisch durch Demethylierung von Galanthamin herzustellen.

So wird in der WO-A1-9703987 eine zweistufige Demethylierung von Galanthamin durch Abbau des N-Oxids beschrieben.

Weiters wird in Tetrahedron Lett. 1997, 38, 5151, Mary, Aude; Renko, Dolor Zafiarisoa; Guillou, Catherine; Thal, Claude "Selective N-demethylation of galanthamine to norgalanthamine via a non classical Polonovski reaction" eine zweistufige Demethylierung von Galanthamin durch Umsetzen mit Kohlensäurederivaten beschrieben.

Die zweistufige Demethylierung von Galanthamin durch Umsetzen mit Azodicarbonsäureestern und anschließender Verseifung ist aus der US-A-5 958 903 vorbekannt.

Weiters stellt die N-Demethylierung eine klassische Reaktion der Alkoloidchemie dar, wobei insbesondere N-Dealkylierungen mit folgenden Reagentien bekannt sind:
- BrCN/Zn
   Seiler, Max. P.; Hagenbach, Alexander; Wuethrich, Hans-Juerg; Markstein, Rudolf. J. Med. Chem. **1991,** *34*, 303 - 307
- Chlorethylchloroformiat
   Ghosh, Debasis; Snyder, Scott E.; Watts, Val J.; Mailman, Richard B.; Nichols, David E. J. Med. Chem. **1996,** *39,* 549 - 555
- NaOCl, Phasentransferkatalyse, dann NaOH
   Robson, Claire; Meek, Michelle A.; Grunwaldt, Jan-Dierk; Lambert, Peter A.; Queener, Sherry F. J. Med. Chem. **1997,** *40*, 3040 - 3048
- N-Iodsuccinimid
   Stenmark, Heather G.; Brazzale, Antony; Ma, Zhenkun. J. Org. Chem. **2000,** *65*, 3875 - 3876
- Chlorameisensäuremethylester
   Kim, J. C. Org. Prep. Proced. Int. **1977,** 9, 1
- Palladium auf Aktivkohle/O₂
   Chaudhuri, Naba K.; Servando, Ofelia; Markus, Bohdan; Galynker, Igor; Sung, Ming-Sang. J. Indian Chem. Soc. **1985,** *62*, 899 - 903.
- Chlorameisensäureethylester
   Humber, L. G.; Charest, M. P.; Herr, F. J. Med. Chem. **1971,** *14*, 982
- Pyridinhydrochlorid
   Radl, Stanislov. **Pyridine hydrochloride in organic synthesis.** Janssen Chim. Acta **1989,** *7*, 12- 17
- Zahlreiche Chloroformiate
   Olofson, R. A.; Martz, Jonathan T.; Senet, Jean Pierre; Piteau, Marc; Malfroot, Thierry. A new reagent for the selective, high-yield N-dealkylation of tertiary amines: improved syntheses of naltrexone and nalbuphine. J. Org. Chem. 1984, 49, 2081 - 2082
- BrCN/Hydrolyse
   Lee, Shoei Sheng; Liu, Yi Chu; Chang, Shu Hwei; Chen, Chung Hsiung. **N-Demethylation studies of pavine alkaloids.** Heterocycles **1993**,*36*. 1971 - 1974
- Fe/Fe²⁺/O₂
   Sparfel, Daniel; Baranne-Lafont, Joele; Nguyen Kim Cuong; Capdevielle, Patrice; Maumy, Michel. **II. Catalytic oxidation** of **2-(aminomethyl)phenols with the system ferrous chloride-Iron-oxygen.** Tetrahedron **1990,** *46*, 803-814

Ein Verfahren zur oxidativen Demethylierung unter Verwendung von Eisensulfat ist zwar aus der WO-A-01/74820 bekannt, jedoch musste hier das gewonnene Rohprodukt relativ aufwendig gereinigt werden.

Diese bekannten Verfahren haben den Nachteil, dass sie auf Grund ihrer niedrigen Ausbeuten, der relativ teuren Ausgangsprodukte sowie auf Grund der mitunter gefährlichen Reaktionsbedingungen im Industriemaßstab nur schwer durchführbar sind.

Aufgabe der vorliegenden Erfindung ist es daher, ein Verfahren der eingangs genannten Art anzugeben, welches auch im Industriemaßstab wirtschaftlich durchführbar ist und mit welchem gleichzeitig Verfahrensendprodukte hoher Reinheit erzeugt werden können.

Die Erfindung betrifft ein Verfahren zur Herstellung von Salzen mit der allgemeinen Formel (B) 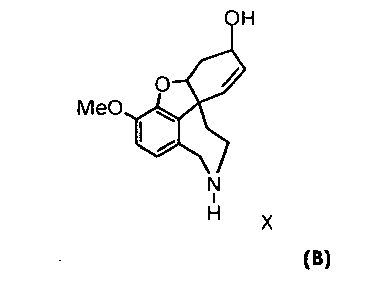 wobei X gleich HCL oder Oxalsäure ist, durch oxidative Demethylierung der entsprechenden Galanthaminverbindungen mit der allgemeinen Formel (1) 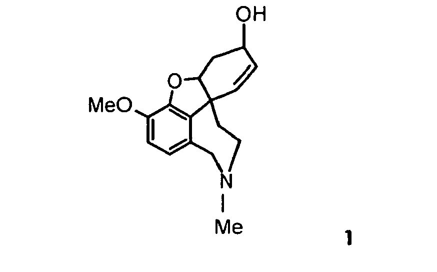 unter Bildung eines N-Oxid-Zwischenproduktes mit der allgemeinen Formel (2) 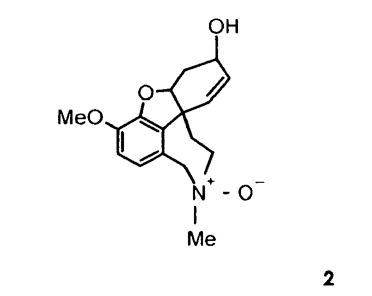 aus welchem durch Zugabe von Eisensulfat in 0,1 bis 2,0 äquimolarer Menge sowie Zugabe von Chlorwasserstoff oder Oxalsäure und anschließendem Umkristallisieren aus einem oder mehreren Lösungsmitteln Verbindungen der Formel (B) gebildet werden.

Weitere vorteilhafte Ausgestaltungen des erfindungsgemäßen Verfahrens sind gemäß Unteransprüche offenbart. Das erfindungsgemäße Verfahren lässt sich anhand des folgenden Syntheseschemas erläutern:

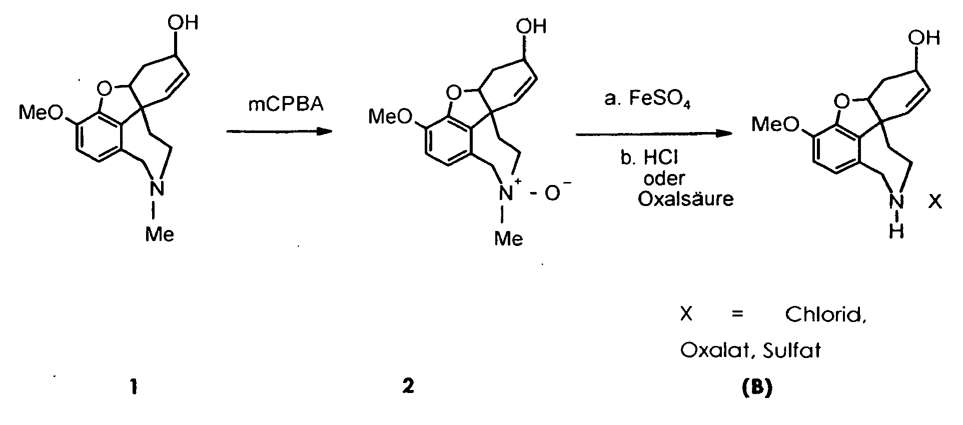

Dabei werden die jeweiligen Galanthaminverbindungen mit der allgemeinen Formel (1) durch Oxidation in das Zwischenprodukt mit der allgemeinen Formel (2), das sogenannte N-Oxid-Zwischenprodukt umgesetzt.

Als Oxidationsmittel eignen sich beispielsweise Wasserstoffperoxyd oder substituierte Perbenzoesäuren. Besonders bevorzugt ist m-Chlorperoxybenzoesäure (mCPBA).

Anschließend wird das leicht abtrennbare Norgalanthamin-Salz gemäß allgemeiner Formel (B) beispielsweise als Chlorid, Oxalat oder Sulfat erzeugt und aus dem Reaktionsgemisch durch Fällung abgetrennt. Das abgetrennte Salz besitzt bereits hohe HPLC-Reinheit, kann jedoch durch weiteres Umkristallisieren zusätzlich gereinigt werden. Insgesamt ist das Verfahren auch zur Durchführung im Industriemaßstab geeignet, da ein Reinigungsschritt mittels Salzfällung auch im großen Maßstab nur einen relativ geringen Aufwand darstellt.

Vorteilhafte erfindungsgemäße Verfahrensvarianten werden anhand der folgenden, beispielhaften Ausführungen näher erläutert:

### Beispiel 1:

### Herstellung von Norgalanthamin Hydrochlorid (B, X=HCl)

2,10 kg (7,3 mol) (-)-Galanthamin werden in 12,6 I Chloroform unter Rühren gelöst. In dieser Lösung werden 1,93 kg (8,5 mol) m-Chlorperoxybenzoesäure (mCPBA) (75,6 %ig) während eines Zeitraums von 30-120 min portionsweise so zugegeben, dass die Temperatur der Lösung zwischen 15°C und 50°C liegt. Nach ein- bis zweistündigem Rühren ohne Temperaturregelung wird das Reaktionsgemisch auf 0-10 °C abgekühlt. Es wird nun eine Lösung von 0,20 kg (0,73 mol) Eisen(II)sulfat heptahydrat in 7,30 l Wasser während eines Zeitraums von 40-50 min so zugetropft, dass die Temperatur des Reaktionsgemisches zwischen 0 °C und 10 °C bleibt. Nach vollständiger Zugabe wird das Reaktionsgemisch noch 10 min bei 0-10 °C rühren gelassen. Nach Zugabe von 1,05 l konz. Salzsäure werden anschließend 10,0 l Chloroform im Vakuum abdestilliert. Das Reaktionsgemisch wird dreimal mit je 9,40 l MTBE bei 30 °C bis 40 °C extrahiert um die Benzoesäure zu entfernen. Die wässrige Phase wird 5 Stunden bis 24 Stunden bei 0 °C bis 10 °C gerührt. Der entstandene Niederschlag wird abgesaugt, mit 2,0 I MTBE gewaschen und bei 50 °C im Vakuum getrocknet.
Ausbeute: 1,87 kg (80,5 % d. Th.)
HPLC Reinheit 95,4 %
Schmp. 173-180°C; ¹H NMR (DMSO-d₆): δ 9.70 (b, 1.5 H), 6,92-6.79 (m, 2H), 6.15 (d, 1H), 5.87 (m, 1H), 4.50 (m, 1H), 4.48-4.03 (m, 5H), 3.75 (s, 3H), 2.50-1.80 (m, 5H): ¹³C NMR (DMSO-d₆): δ 147.36 s, 145.37 s, 133.67 s, 130.30 d, 126.55 d, 123.33 s, 122.76 d, 112.69 d, 87.24 d, 60.33 d, 56.54 q, 49.97 t, 48.03 s, 45.14 t, 35.08 t, 31.63 t.

### Beispiel 2:

### Herstellung von Norgalanthamin Oxalat (B, X=Oxalsäure)

Verwendet man anstelle von HCl, wie in Beispiel 1, Oxalsäure-Dihydrat, so lässt sich das gebildete Oxalatsalz des Norgalanthamins isolieren.
Ausbeute: 75 % d. Th.
HPLC Reinheit 96 %
Schmp. 233 - 235°C; ¹H NMR (DMSO-d₆): δ 6.85 - 6.75 (m, 2H), 6.11 (d, *J* = 10.31 Hz, 1H), 5.86 (dd, *J* = 10.31, 4.4 Hz, 1H), 4.54 (b, 1H), 4.47 (d, *J* = 15.7 Hz, 1H), 4.24 (d, *J* = 15.1 Hz, 1H), 4.09 (b,1H), 3.79 (s, 3H), 3.55 - 3.40 (m, 2H), 2.54 - 2.46 (m, 1 H), 2.35 - 2.20 (m, 1 H), 2.16 - 1.82 (m, 3H); ¹³C NMR (DMSO-d₆): δ 164.9 (s), 146.5 (s), 144.5 (s), 132.9 (s), 129.5 (d), 125.7 (d), 122.8 (s), 121.7 (d), 111.7 (d), 86.4 (d), 59.6 (d), 55.7 (q), 49.4 (t), 47.2 (s), 44.4 (t), 34.4 (t), 30.8 (t); Anal. ber. für C₁₆H₁₉NO₃*C₂H₂O₄*0.5 H₂O: C, 58.06; H, 5.95; N, 3.76. Gefunden: C, 57.91; H, 5.88; N, 3.69.

### Beispiel 3:

### Reinigung von Norgalanthamin-Hydrochlorid (B, X=HCl):

5,20 kg (16,8 mol) (-)-Norgalanthamin-HCl werden als Rohprodukt in 10,4 l Wasser aufgenommen und unter Rühren bei Rückflußtemperatur gelöst. Nach 10-15 min Rühren bei Rückflusstemperatur wurde auf 0-10 °C abgekühlt und 1 bis 24 Stunden bei dieser Temperatur gerührt. Der Niederschlag wird abgesaugt und mit 1,0 l Wasser gewaschen und bei 50 °C 80-120 h im Vakuum getrocknet.
Ausbeute: 3,96 kg (76,2 % d. Th.)
HPLC Reinheit 98,9 %

Im folgenden wird ein Vergleich mit dem nächstliegenden Stand der Technik, das ist die Lehre gemäß der WO-A1-9703987, durchgeführt.

### 1) Herstellung der N-Oxid-Zwischenverbindung (2):

In der WO-A1-9703987 wird die N-Oxid-Bildung als Standardverfahren erwähnt. Ohne detaillierte Angabe darf angenommen werden, dass dieses Zwischenprodukt hergestellt und anschließend - wie im folgenden dargestellt - einer aufwendigen Reinigung unterzogen wird.

### 2) Herstellung der Verbindung B mittels FeSO₄ - Reduktion aus (2):

| Parameter | WO-A1-9703987 | Sanochemia Verfahren |
|---|---|---|
| Ansatzgröße | 1,73 g | 2,1 kg |
| Lösungsmittelmenge | ca. 60fach | ca. 10fach |
| eq.Eisensulfat heptahydrat | 2 | 0,1 |
| Aufarbeitung | Eindampfen zur Trockene, Extraktion zwischen Methylenchlorid und NaHCO₃-Lösung | Versetzen mit HCl, Einengen auf 50% und Extraktion zwischen Wasser und MTBE |
| Isolation des Produktes | als freie Base durch Eindampfen zur Trockene | HCl-Salz durch Abkühlen, und Absaugen der wässrigen Phase |
| Reinigung | Säulenchromatographie | Umkristallisation |
| Endprodukt | freie Base | HCl- oder Oxalat-Salz |

Vorteile des erfindungsgemäßen Verfahrens gegenüber dem Verfahren aus der WO-Al-9703987:

Das Reaktionsvolumen ist durch Verwenden von einem Sechstel (1/6) an Lösungsmittelmenge im Vergleich zur WO-A1-9703987 deutlich geringer.

Durch das Verwenden von nur 5% Eisensulfatheptahydrat sind im Vergleich mit der gegenüber der WO-A1-9703987 deutlich niedrigere Eisenrückstände im Reaktionsgemisch enthalten.

Beim Aufarbeiten wird das Reaktionsgemisch auf 50% des Gesamtvolumens eingeengt. Ein Eindampfen zur Trockene, wie dies in der WO-A1-9703987 sogar zweimal durchgeführt wird, ist im Industriemaßstab nur schwer möglich.

Das Verfahrensendprodukt mit der allgemeinen Formel (B) wird beispielsweise als kristallines Hydrochlorid-Salz durch Abkühlen aus dem wässrigen Reaktionsgemisch gefällt, wogegen gemäß der WO-A 19703987 ein nur schwer weiterverarbeitbarer "weißer Schaum" gebildet wird.

Eine Aufreinigung des Verfahrensendproduktes gemäß der allgemeinen Formel (B) auf Reinheitsgrade von >98% ist durch einfaches Umkristallisieren aus Wasser möglich. Eine aufwendige säulenchromatographische Reinigung des Verfahrensproduktes ist im Gegensatz zur WO-A1-9703987 jedoch nicht nötig.

Zusammenfassend lässt sich sagen, dass sich das erfindungsgemäße Verfahren aufgrund der niedrigen Eisenrückstände und der reduzierten Lösungsmittelmenge besonders für die Durchführung im Industriemaßstab eignet, wobei jedoch gleichzeitig zufriedenstellende Reinheitsgrade erzielt werden.

## Patentansprüche

1. Verfahren zur Herstellung von Salzen des Norgalanthamin, mit der allgemeinen Formel (B) 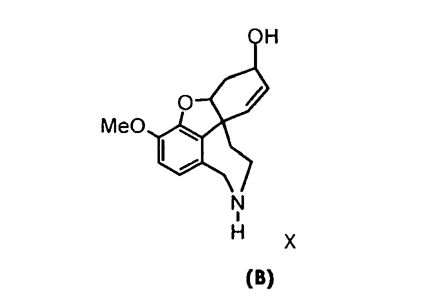 wobei X gleich HCl oder Oxalsäure ist, durch oxidative Demethylierung der entsprechenden Galanthaminverbindungen mit der allgemeinen Formel (1) 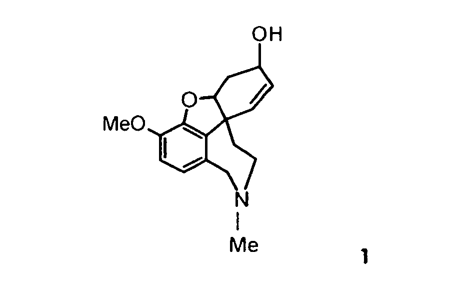 unter Bildung eines N-Oxid-Zwischenproduktes mit der allgemeinen Formel (2) 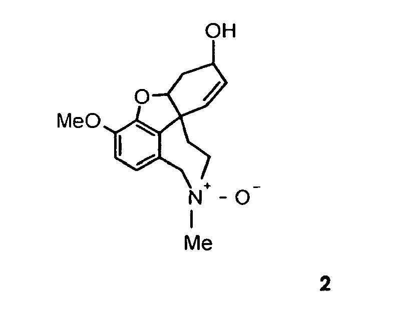 aus welchem durch Zugabe von Eisensulfat in 0,1 bis 2,0 äquimolarer Menge sowie Zugabe von Chlorwasserstoff oder Oxalsäure und anschließendem Umkristallisieren aus einem oder mehreren Lösungsmitteln Verbindungen der Formel (B) gebildet werden.

2. Verfahren nach Anspruch 1, **dadurch gekennzeichnet, dass** als Eisensulfat ein Eisensulfatheptahydrat eingesetzt wird.

3. Verfahren nach Anspruch 1 oder 2, **dadurch gekennzeichnet, daß** für die oxidative Demethylierung als Oxidationsmittel Peroxide eingesetzt werden.

4. Verfahren nach Patentanspruch 3, **dadurch gekennzeichnet, daß** als Peroxide substituierte und/oder unsubstituierte Perbenzoesäuren, insbesondere m-Chloroxybenzoesäure (mCPBA) eingesetzt werden.

## Claims

1. Process for the production of norgalanthamine salts with general formula (B), 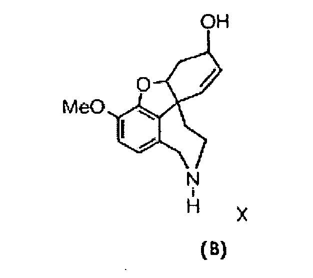 wherein X stands for HC1 or oxalic acid by oxidative demethylation of the corresponding galanthamine compounds with general formula (1) 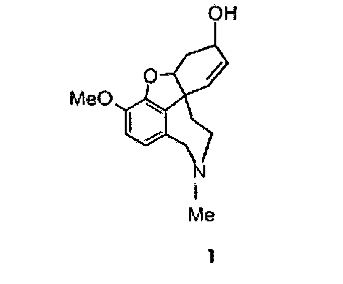 by forming an N-oxide intermediate product with general formula (2) 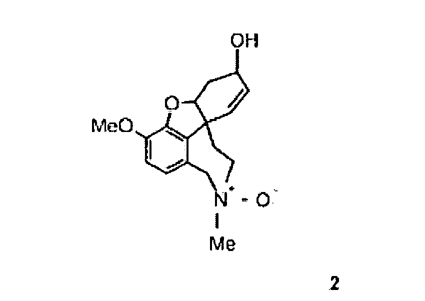 from which the compounds of formula (B) are formed by addition of iron sulfate in a 0.1 to 2.0 equimolar amount and addition of hydrochloric acid and oxalic acid and following recrystallization from one or more solvents.

2. Process according to claim 1, **characterized in that** iron sulfate heptahydrate is used as iron sulfate.

3. Process according to claim 1 or 2, **characterized in that** peroxides are used as oxidizing agents for the oxidative demethylation.

4. Process according to claim 3, **characterized in that** substituted and/or unsubstituted perbenzoic acids, in particular m-chloroxybenzoic acid (mCPBA) are used as peroxides.

## Revendications

1. Procédé de fabrication de sels de norgalanthamine, ayant la formule générale (B) 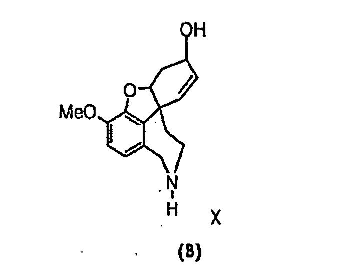 dans laquelle X est égal à HCl ou l'acide oxalique, par déméthylation oxydative des composés de galanthamine correspondants avec la formule générale (1) 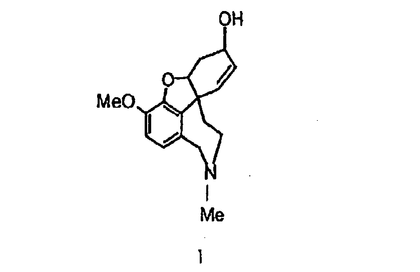 en formant un produit intermédiaire de N-oxyde ayant la formule générale (2) 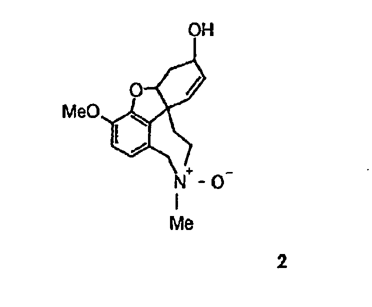 à partir duquel il se forme des composés de formule (B) par addition de sulfate de fer en quantité de 0,1 à 2,0 équimolaire et addition de chlorure d'hydrogène ou d'acide oxalique et recristallisation consécutive à partir d'un ou de plusieurs solvants.

2. Procédé selon la revendication 1, **caractérisé en ce que** l'on met en oeuvre à titre de sulfate de fer un heptahydrate de sulfate de fer.

3. Procédé selon la revendication 1 ou 2, **caractérisé en ce que**, pour la déméthylation oxydative, on met en oeuvre comme agents d'oxydation des peroxydes.

4. Procédé selon la revendication 3, **caractérisé en ce que** l'on met en oeuvre, à titre de peroxydes, des acides perbenzoiques substitués et/ou non substitués, en particulier de l'acide m-chloroxybenzoïque (mCPBA).
